# FASCICULE DE BREVET EUROPEEN

(11) **EP 4 444 376 B1**
(45) Date de publication et mention de la délivrance du brevet: **08.04.2026**
(21) Numéro de dépôt: 22834667.2
(22) Date de dépôt: 04.11.2022
(51) Int. Cl.: A61M 1/00, C12M 1/00, C12M 1/33

(54) **DISPOSITIF DE FILTRATION À USAGE UNIQUE POUR LA PURIFICATION DE TISSU ADIPEUX**
EINWEG-FILTRATIONSVORRICHTUNG ZUR REINIGUNG VON FETTGEWEBE
DISPOSABLE FILTRATION DEVICE FOR PURIFYING ADIPOSE TISSUE

(30) Priorité: 08.12.2021 FR 2113140
(43) Date de publication de la demande: 16.10.2024
(73) Titulaire: Neosyad, 13290 Aix-en-Provence (FR)
(72) Inventeur: ROCHE, Régis, 83510 Lorgues (FR); CABAUD, François, 13510 Eguilles (FR); NELISSEN, Xavier, 4453 Villers Saint Siméon (BE)
(74) Mandataire: Cabinet Beau de Loménie
(86) Numéro de dépôt international: PCT/FR2022/052078
(87) Numéro de publication internationale: WO 2023/105130

(56) Documents cités:
- EP-A1- 3 650 531
- US-A1- 2018 221 550
- US-A1- 2019 247 557
- US-A1- 2020 061 259

## Description

### Domaine Technique

La présente invention concerne un dispositif de filtration à usage unique pour la purification de tissu adipeux. L'invention trouve notamment une application pour les opérations de transplantation de graisse (désignées par « lipofilling » en langue anglaise) à visées esthétiques et reconstructrices. L'invention trouve, en particulier, une utilisation pour la transplantation de graisse utile pour les opérations de chirurgie mammaire, la mise en œuvre du dispositif objet de l'invention n'étant toutefois pas limitée à cette application.

### Technique antérieure

Des opérations de transplantation de graisse ou de greffe autologue de tissu adipeux sont utilisées en chirurgie notamment en chirurgie du fessier pour une augmentation de volume ou en chirurgie mammaire afin de modeler le sein pour lui donner un aspect plus naturel après un DIEP (pour « Deep Inferior Epigastric Perforator » ou Perforante de l'artère épigastrique profonde intérieure), une reconstruction par lambeau de grand dorsal, ou après le placement d'un implant mammaire. Ces opérations consistent à prélever de la graisse d'un patient dans une zone donneuse pour la réintroduire ensuite dans la zone d'intérêt du corps du patient.

Ces opérations n'ont actuellement pas un rendu reproductible et peuvent nécessiter plusieurs interventions du fait de la résorption de la matière introduite (perte de volume rencontrée après l'opération). En effet, le tissu adipeux prélevé contient un milieu liquide constitué principalement, d'une part, d'huile et de sang présents initialement dans le tissu adipeux et, d'autre part, d'un liquide tel qu'une solution physiologique introduite lors du prélèvement du tissu adipeux et/ou lors d'une opération de lavage de celui-ci.

Les inventeurs ont déterminé que le taux de résorption est en grande partie lié à la quantité de liquide présente dans le tissu adipeux réintroduit dans le corps du patient. En d'autres termes, plus il reste de liquide dans le tissu adipeux réinjecté, plus le taux de résorption est élevé. Il est donc important de traiter le tissu adipeux avant sa réintroduction dans le corps du patient afin de retirer un maximum de milieu liquide. Ce traitement doit néanmoins être réalisé dans des conditions d'hygiène contrôlées afin d'éliminer les risques de contamination d'un patient à l'autre.

Le document US 2020/0054824 divulgue un système de transplantation de graisse qui utilise un dispositif de filtration par gravité commercialisé sous la référence commerciale Revolve^{®}. Cependant, ce type de dispositif de filtration ne permet pas de retirer du tissu adipeux une quantité suffisante de liquide apte à diminuer le taux de résorption de manière significative. Par ailleurs, dans le système du document US 2020/0054824 le tissu adipeux circule dans un circuit de pompage qui doit être désinfecté entre deux opérations, ce qui entraîne une immobilisation du système après chaque transplantation. Il est en outre difficile de contrôler la désinfection dans l'ensemble du circuit de pompage.

Or, il existe un besoin pour une solution permettant de réaliser un traitement optimal d'élimination du milieu liquide présent dans un tissu adipeux tout en assurant de très bonnes conditions d'hygiène.

### Exposé de l'invention

A cet effet, la présente invention propose un dispositif de filtration à usage unique pour la purification d'un tissu adipeux comprenant au moins :
- une enceinte étanche,
- un filtre présent dans l'enceinte étanche, ledit filtre délimitant une chambre de centrifugation pour un tissu adipeux,
- des tuyaux reliés à l'enceinte étanche,
- un plateau rotatif en liaison avec le filtre, le plateau rotatif étant configuré pour coopérer avec un dispositif d'entraînement en rotation.

Le dispositif de filtration de l'invention combine ainsi avantageusement l'utilisation d'un filtre apte à drainer un milieu liquide d'un tissu adipeux avec la mise en rotation de celui-ci. En effet, le filtre formant une chambre de centrifugation, un tissu adipeux présent à l'intérieur de cette chambre peut être soumis à des accélérations centrifuges contre la paroi interne du filtre. Le milieu liquide présent dans et autour du tissu adipeux est alors efficacement drainé en dehors de la chambre de centrifugation à travers les pores du filtre alors que le tissu adipeux est retenu dans la chambre. On élimine ainsi une quantité importante de liquide permettant de disposer d'un tissu adipeux purifié qui, une fois réimplanté, présente un taux de résorption bien inférieur à celui obtenu avec les solutions de filtration de l'art antérieur.

Par ailleurs, le dispositif de filtration de l'invention comprend des tuyaux utilisés pour la circulation du tissu adipeux. Les tuyaux faisant partie du dispositif à usage unique, on assure ainsi une parfaite hygiène à chaque intervention par le remplacement des éléments dans lesquels circule ou transite le tissu adipeux.

Dans un exemple de réalisation, le dispositif de purification comprend en outre un élément de rigidification présent entre l'enceinte étanche et le filtre.

Dans un autre exemple de réalisation, le filtre est en un matériau rigide autoporteur. Dans ce cas, un élément de rigidification n'est plus nécessaire dans le dispositif de purification.

Selon l'invention telle que revendiquée, le dispositif de purification comprend en outre un plateau de collecte présent dans la chambre de centrifugation, le plateau de collecte étant mobile en translation suivant l'axe de rotation du filtre. Le plateau de collecte joue un rôle de piston qui permet de racler la paroi interne du filtre de manière à collecter un maximum de tissu adipeux et de faciliter le prélèvement du tissu adipeux purifié en sommet du dispositif.

Selon un autre aspect particulier, le dispositif de purification comprend en outre une tige filetée s'étendant dans la chambre de centrifugation et coopérant avec une portion taraudée du plateau de collecte, la tige filetée étant reliée au moyen d'entrainement en rotation du filtre, ledit moyen étant configuré pour entraîner la tige filetée en rotation suivant un sens de rotation opposé au sens de rotation du filtre. On utilise ici avantageusement le même moyen d'entrainement en rotation à la fois pour la mise en centrifugation du filtre et le déplacement en translation verticale du plateau de collecte.

Selon un autre aspect particulier, le dispositif de purification comprend en outre une gaine de protection entourant la tige filetée. Cette gaine permet d'éviter à la tige filetée d'entrer en contact avec le tissu adipeux qui en s'accumulant au niveau du taraudage du plateau peut bloquer le déplacement de celui-ci.

L'invention a également pour objet une machine pour la purification d'un tissu adipeux d'un patient, la machine comprenant au moins :
- un dispositif de filtration à usage unique selon l'invention,
- un dispositif d'entraînement en rotation sur lequel le dispositif de filtration à usage unique est fixé de façon amovible, le dispositif d'entraînement en rotation comprenant un moteur rotatif relié au dispositif de filtration à usage unique,
- une pompe d'aspiration reliée au dispositif de filtration à usage unique,
- une pompe de circulation reliée au dispositif de filtration à usage unique,
- un réservoir de liquide de lavage relié au dispositif de filtration à usage unique,
- une unité de commande configurée pour commander le dispositif d'entraînement en rotation, la pompe d'aspiration et la pompe de circulation.

La machine de l'invention permet de réaliser toutes les opérations nécessaires (prélèvement, lavage, centrifugation et réimplantation de tissu adipeux) à un traitement de transplantation de tissu adipeux, et ce dans de très bonnes conditions d'hygiène à chaque traitement grâce à l'utilisation d'un dispositif de filtration à usage unique.

Dans un exemple de réalisation, la machine comprend en outre un dispositif de vannes à pincement coopérant avec des tuyaux du dispositif de filtration à usage unique, l'unité de commande étant configurée pour commander ledit dispositif de vanne à pincement de manière à empêcher sélectivement la circulation dans un ou plusieurs tuyaux.

Le dispositif de vannes à pincement permet de contrôler sélectivement la circulation dans les tuyaux tout en facilitant le montage et le démontage des tuyaux lors du remplacement du dispositif de filtration à usage unique.

Dans un exemple de réalisation, le dispositif d'entraînement en rotation comprend une bride stator, l'enceinte étanche du dispositif de filtration à usage unique comprenant des moyens de fixation amovible aptes à bloquer en rotation ladite enceinte étanche sur la bride stator.

Il est ainsi possible de verrouiller et déverrouiller rapidement et facilement un dispositif de filtration à usage unique sur la machine.

Brève description des dessins
[Fig. 1] La figure 1 est vue schématique en perspective d'une machine pour la purification d'un tissu adipeux conformément à un mode de réalisation de l'invention.
[Fig. 2] La figure 2 est une vue schématique éclatée d'un dispositif de filtration à usage unique conformément à un exemple de réalisation de l'invention,
[Fig. 3] La figure 3 est une vue schématique en coupe du dispositif de filtration à usage unique de la figure 1 une fois assemblé,
[Fig. 4] La figure 4 est une vue schématique en coupe montrant le dispositif de filtration à usage unique de la figure 2 après déplacement du plateau de collecte,
[Fig. 5] La figure 5 est une vue schématique en coupe montrant le dispositif de filtration à usage unique de la figure 2 avant fixation sur un dispositif d'entraînement en rotation de la machine pour la purification d'un tissu adipeux,
[Fig. 6] La figure 6 est une vue schématique en coupe montrant le dispositif de filtration à usage unique de la figure 2 après fixation sur un dispositif d'entraînement en rotation de la machine pour la purification d'un tissu adipeux,
[Fig. 7] La figure 7 est une vue de détail d'un dispositif de vannes à pincement de la machine pour la purification d'un tissu adipeux de la figure 1.

### Description des modes de réalisation

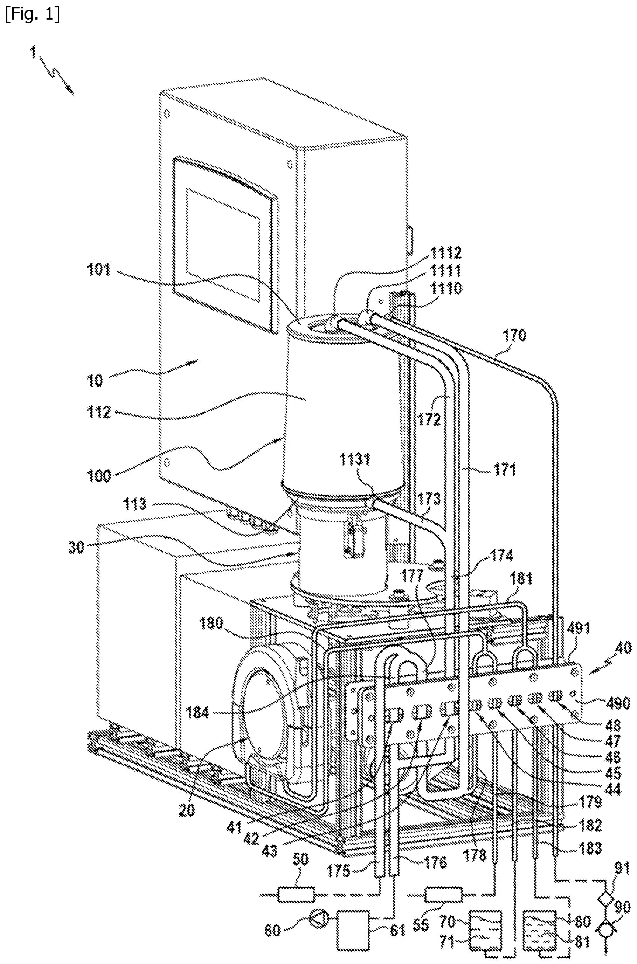

La figure 1 illustre un exemple de machine 1 selon l'invention pour la purification d'un tissu adipeux. D'une manière générale et comme il sera davantage détaillé plus bas, la machine 1 comprend une unité de commande 10 permettant la mise en œuvre des différentes étapes de traitement, une pompe de circulation 20 et une pompe d'aspiration 60 pilotées par l'unité de commande 10, un dispositif d'entraînement en rotation 30 piloté par l'unité de commande 10.

La machine 1 comprend en outre un dispositif de filtration à usage unique 100 pour la purification d'un tissu adipeux conformément à un mode de réalisation de l'invention, le dispositif de filtration 100 étant monté de manière amovible sur la machine 1.

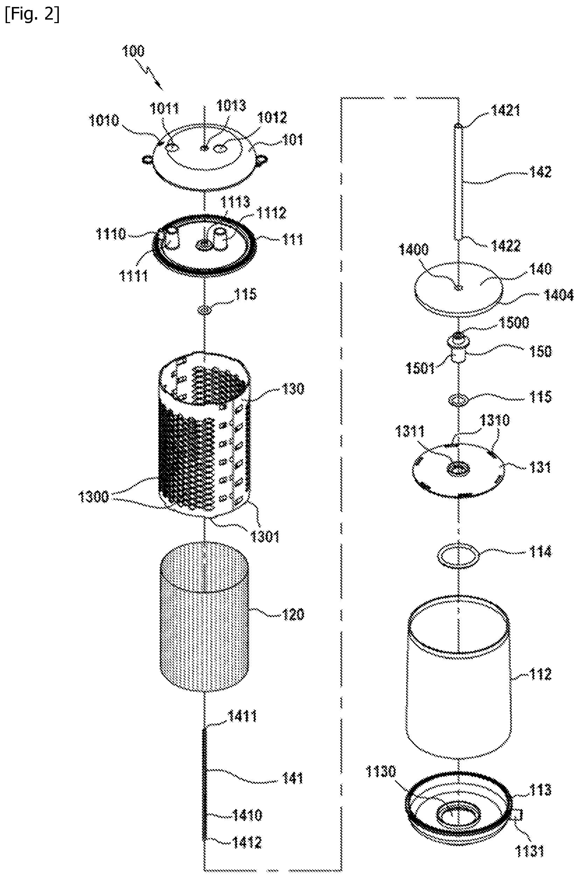

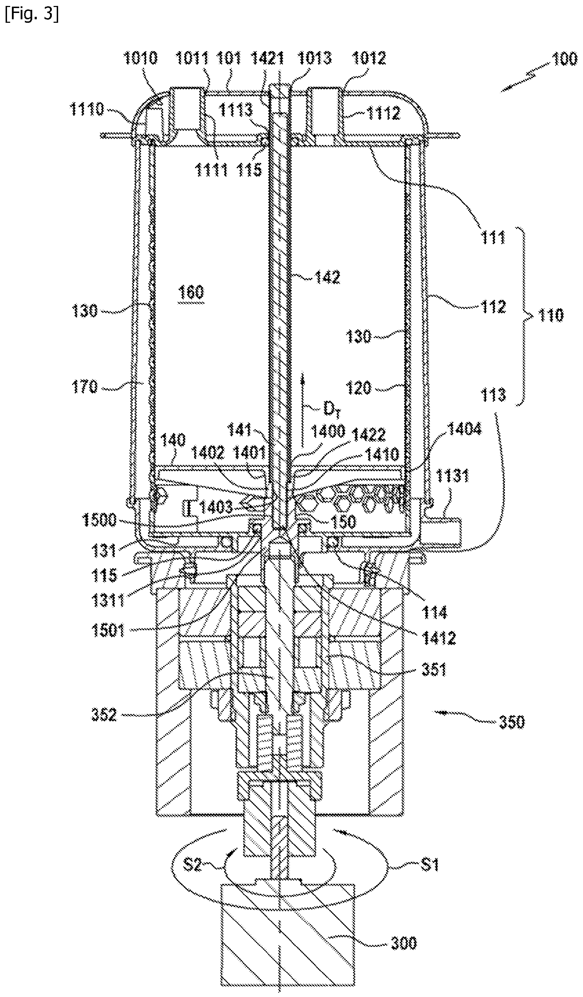

Comme illustré sur les figures 2 et 3, le dispositif de filtration à usage unique 100 comprend notamment :
- une enceinte étanche 110,
- un filtre 120 présent dans l'enceinte étanche 110, ledit filtre délimitant une chambre de centrifugation 160 pour un tissu adipeux,
- plusieurs tuyaux 170 à 173 reliés à l'enceinte étanche 110,
- un plateau rotatif 131 en liaison avec le filtre 120, le plateau rotatif étant configuré pour coopérer avec le dispositif d'entraînement en rotation 30.

L'enceinte étanche 110 est formée ici par un capot 111, une paroi cylindrique 112 et un fond 113. Ces éléments sont fixés ensemble de manière étanche.

Le dispositif de filtration 100 comprend également un filtre ou crible 120 présent dans l'enceinte étanche 110. Le filtre 120 délimite une chambre de centrifugation 160 (figure 3) dont le fonctionnement est décrit plus loin. Dans l'exemple décrit ici le filtre 120 présente une forme cylindrique. Le filtre peut présenter d'autres formes adaptées pour la centrifugation. Le filtre 120 présente une taille de pore configurée pour laisser passer un milieu liquide et retenir un tissu adipeux. La taille de pore est choisie en particulier pour permettre le passage de liquides tels que de l'huile, du sang, de l'eau ou une solution physiologique tout en retenant le tissu adipeux. A titre d'exemples non limitatifs, la taille de pore du filtre peut être comprise entre 50 µm et 1500 µm et plus préférentiellement entre 200 µm et 500 µm. Le filtre peut être réalisé de différentes manières comme par exemple par tressage, soudage de fils de plastique ou à partir d'un matériau comprenant des ajours ou trous ayant des dimensions correspondant à la taille de pore souhaitée. Le filtre peut être notamment en un matériau polymérique tel que du polyester ou du polypropylène mais l'homme du métier reconnaîtra que d'autres matériaux peuvent être utilisés. Dans l'exemple décrit ici, un élément de rigidification 130 de forme cylindrique est présent entre la paroi cylindrique 112 de l'enceinte étanche 110 et le filtre 120. Il a notamment pour fonction d'assurer la tenue structurale du filtre 120 pendant la centrifugation. L'élément de rigidification 130 est par exemple réalisé en matériau métallique ou plastique et présente une structure ajourée définissant une pluralité d'ouvertures 1300 afin de permettre l'évacuation du milieu liquide drainé par le filtre 120.

L'élément de rigidification 130 est associé à un plateau rotatif 131. Plus précisément, l'élément de rigidification 130 comporte à son extrémité inférieure des dents 1301 qui coopèrent avec des rainures 1310 présente au voisinage de la périphérie externe du plateau rotatif 131. Bien entendu, tout autre moyen de solidarisation de l'élément de rigidification avec le plateau rotatif, comme par exemple un clipsage ou collage, peut être envisagé. Le plateau rotatif 131 est relié au dispositif d'entraînement en rotation 30. Le dispositif d'entraînement en rotation comprend un moteur électrique rotatif 300. Le moteur électrique 300 peut être par exemple un moteur pas à pas ou un moteur à courant continu sans balai. Dans l'exemple décrit ici, le plateau rotatif 131 est relié au moteur électrique rotatif 300 par un embrayage bidirectionnel 350 configuré pour assurer, via un premier arbre 351, l'entrainement en rotation du plateau rotatif 131 suivant un premier sens de rotation S₁ du moteur 30 (figure 3). L'embrayage bidirectionnel peut être remplacé par tout dispositif permettant un entraînement en rotation sélectif suivant deux sens de rotation opposés. Des joints 114 et 115 sont placés respectivement en-dessous et au-dessus du plateau rotatif 131 afin d'assurer l'étanchéité dans la partie inférieure du dispositif de purification. Le dispositif de filtration 100 étant à usage unique, il est monté de manière interchangeable sur la machine. A cet effet et comme illustré sur les figures 3 à 6, le dispositif d'entraînement en rotation 30 comprend une bride stator 310 sur laquelle est fixée le fond 113 de l'enceinte étanche 110. Dans l'exemple décrit ici, on utilise une fixation de type à baïonnette qui permet à la fois de solidariser le dispositif de filtration à usage unique 100 avec la bride stator 310 du dispositif d'entraînement en rotation 30 de la machine 1 et de bloquer en rotation l'enceinte étanche 110 du dispositif. Plus précisément, le fond 113 de l'enceinte étanche 110 comprend un anneau de fixation 1130 qui présente deux encoches 1133 et 1134 qui coopèrent avec deux tenons 311 et 312 présents sur la bride stator 310. Il est ainsi possible de verrouiller et déverrouiller rapidement et facilement un dispositif de filtration à usage unique 100 sur la machine 1. Tout autre système de fixation amovible peut être utilisé pour solidariser le dispositif de filtration à usage unique sur le dispositif d'entraînement en rotation 30 et bloquer en rotation l'enceinte étanche 110 du dispositif de filtration à usage unique 100.

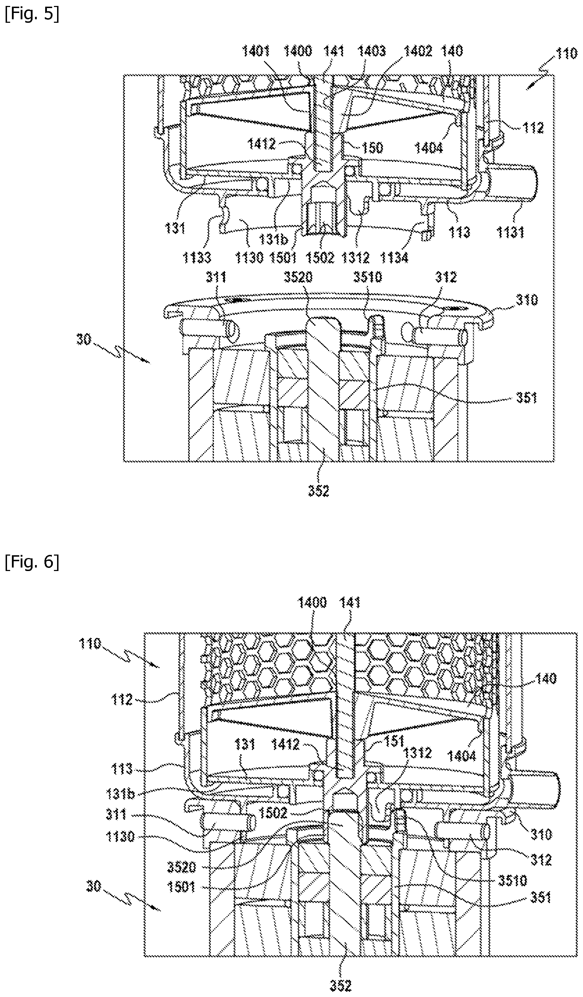

Dans l'exemple décrit ici, le plateau rotatif 131 comprend un doigt 1312 qui s'étend à partir de la face inférieure 131b du plateau rotatif et qui est destiné à coopérer avec un autre doigt 3510 présent sur l'extrémité du premier arbre 351 relié au moteur rotatif 30 lorsque le dispositif de filtration 100 est monté sur la machine (figure 6). Tout autre système permettant un accouplement du dispositif d'entraînement en rotation 30 avec le plateau rotatif 31 peut être utilisé.

La centrifugation est réalisée par mise en rotation du filtre 120. Plus précisément, le moteur électrique 300 est commandé par l'unité de commande 10 suivant un premier sens de rotation S₁ pour entrainer le plateau rotatif 131 et l'élément de rigidification 130 en prise avec le plateau 131. La mise en rotation du plateau 131 et de l'élément de rigidification 130 entraine la mise en rotation du filtre 120. Le filtre est fixé à l'élément de rigidification par tout type de moyen adapté. A titre d'exemples non limitatifs, le filtre peut être fixé avec un ou plusieurs des moyens suivants : collage, engagement dans des crans de calage présents sur l'élément de rigidification, clipsage. La vitesse du moteur électrique 30 est contrôlée par l'unité de commande 10 de manière à appliquer au tissu adipeux présent dans la chambre de centrifugation une accélération centrifuge. Ainsi, un tissu adipeux présent dans la chambre de centrifugation 160 sera soumis à une force centrifuge contre la paroi interne du filtre 120, ce qui permet de drainer efficacement le milieu liquide présent dans le tissu adipeux sans abimer celui-ci.

Le dispositif de filtration à usage unique comprend au moins quatre tuyaux 170, 171, 172 et 173, les tuyaux 170, 171 et 172 étant reliés respectivement aux ports 1110, 1111, 1112 présents sur le capot 111 de l'enceinte étanche 110 tandis que le tuyau 173 est relié à un port d'évacuation 1131 présent sur le fond 113 de l'enceinte 110. L'autre extrémité du tuyau 170 est reliée à un dispositif de mise à l'air 90 muni d'un filtre 91.

Dans l'exemple décrit ici tel qu'illustré sur la figure 1, d'autres tuyaux sont utilisés afin de former des circuits de circulation dans la machine 1 permettant la réalisation des différentes étapes d'un traitement de purification d'un tissu adipeux.

Plus précisément, dans l'exemple de réalisation décrit ici, la machine 1 comprend en outre :
- un tuyau 174 réunissant les tuyaux 172 et 173 connectés au dispositif de filtration 100,
- un tuyau 175 dont une extrémité est reliée à une canule d'aspiration 50 destinée à aspirer du tissu adipeux sur un patient,
- un tuyau 176 dont une extrémité est reliée à une pompe d'aspiration 60, le tuyau 176 débouchant dans le tuyau 174,
- un tuyau 177 reliant le tuyau 175 et un tuyau 184 au tuyau 171,
- un tuyau 178 reliant les tuyaux 171 et 177 à des tuyaux 180 et 179,
- le tuyau 179 dont une extrémité est reliée à une canule d'injection 55 destinée à délivrer un anesthésiant et un tissu adipeux purifié dans le corps du patient, le tuyau 179 étant relié aux tuyaux 178 et 180,
- le tuyau 180 dont une extrémité est reliée à la pompe de circulation 20, le tuyau 180 étant relié aux tuyaux 178 et 179,
- un tuyau 181 dont une extrémité est reliée à la pompe de circulation 20, le tuyau 181 étant relié à des tuyaux 182 et 183,
- le tuyau 182 dont une extrémité est reliée à un réservoir de liquide de lavage 70, le tuyau 182 étant relié au tuyau 181 et à un tuyau 183,
- le tuyau 183 dont une extrémité est reliée à un réservoir de liquide de lavage et d'anesthésiant 80, le tuyau 183 étant relié aux tuyaux 181 et 182,
- un tuyau 184 reliant les tuyaux 175 et 177 avec le tuyau 174.

Tous les tuyaux décrits ci-avant sont à usage unique.

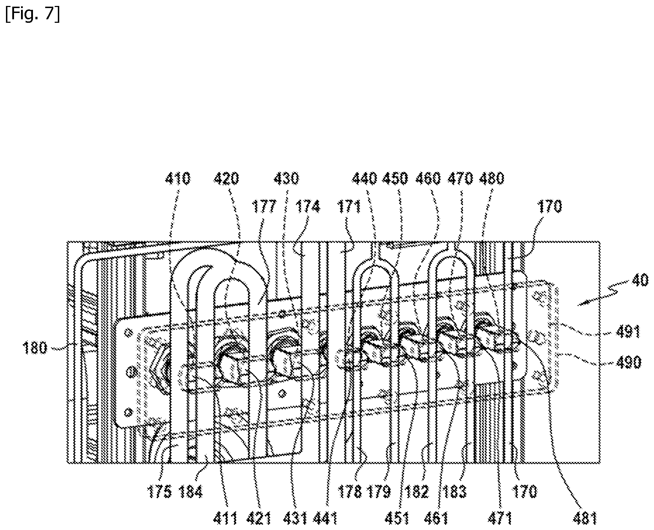

La machine 1 comprend en outre un dispositif de vannes à pincement ou étranglement 40 comprenant une pluralité modules de pincement 41 à 48 encore appelé « pinch valve » aptes à pincer sélectivement un ou plusieurs tuyaux décrits ci-avant afin d'empêcher la circulation et à relâcher le pincement afin d'autoriser cette circulation. Dans l'exemple décrit ici et comme illustré sur la figure 7, les modules de pincement 41 à 48 comprennent chacun un logement 410 à 480 délimité entre deux plaques 490 et 491 et dans lequel est disposé un tuyau. Les modules de pincement 41 à 48 comprennent en outre chacun un doigt 411 à 481 mobile dans le logement correspondant entre une position de retrait (position d'ouverture du module) dans laquelle la circulation est autorisée dans le tuyau présent dans le logement et une position déployée (position de fermeture du module) dans laquelle le doigt pince le tuyau présent dans le logement empêchant ainsi la circulation dans le tuyau.

Dans l'exemple décrit ici :
- le tuyau 184 est présent dans le logement 410 du module de pincement 41,
- le tuyau 177 est présent dans le logement 420 du module de pincement 42,
- le tuyau 174 est présent dans le logement 430 du module de pincement 43,
- le tuyau 178 est présent dans le logement 440 du module de pincement 44,
- le tuyau 179 est présent dans le logement 450 du module de pincement 45,
- le tuyau 182 est présent dans le logement 460 du module de pincement 46,
- le tuyau 183 est présent dans le logement 470 du module de pincement 47,
- le tuyau 170 est présent dans le logement 480 du module de pincement 48.

On décrit maintenant brièvement la position de chaque module de pincement en fonction des étapes ou séquences mises en œuvre par la machine 1.

Dans l'étape d'anesthésie, l'unité de commande 10 place les modules de pincement 45 et 47 dans la position d'ouverture autorisant la circulation dans les tuyaux 179 et 183 et les modules de pincement 41, 42, 43, 44, 46 et 48 dans la position de fermeture empêchant la circulation dans les tuyaux 184, 177, 174, 178, 182 et 170. Par actionnement de la pompe 20 par l'unité de commande 10, un liquide 81 présent dans le réservoir de liquide de lavage et d'anesthésiant 80 est aspiré par le tuyau 183. Le liquide est acheminé dans la pompe 20 par le tuyau 181 puis est refoulé de celle-ci par le tuyau 180 et le tuyau 179 afin d'atteindre la canule d'injection 55 utilisée pour introduire l'anesthésiant dans le corps du patient.

Dans l'étape de prélèvement de tissu adipeux depuis le corps d'un patient, l'unité de commande 10 place les modules de pincement 42 et 43 dans la position d'ouverture autorisant la circulation dans les tuyaux 177 et 174 et les modules de pincement 41, 44, 45, 46, 47 et 48 dans la position de fermeture empêchant la circulation dans les tuyaux 184, 178, 179, 182, 183 et 170. L'actionnement de la pompe d'aspiration 60 par l'unité de commande 10 permet de tirer le vide dans le dispositif de filtration 100 via les tuyaux 176, 174, 173 et 172, ce qui provoque l'aspiration du tissu adipeux par la canule d'aspiration 50 et son acheminement dans le dispositif de filtration 100 via les tuyaux 175, 177 et 171.

Dans l'étape de purification du tissu adipeux prélevé, l'unité de commande 10 place les modules de pincement 44 et 46 dans la position d'ouverture autorisant la circulation dans les tuyaux 178 et 179 et les modules de pincement 41, 42, 43, 45, 47 et 48 dans la position de fermeture empêchant la circulation dans les tuyaux 184, 177, 174, 179, 183 et 170. Par actionnement de la pompe 20 par l'unité de commande 10, un liquide 71 présent dans le réservoir de liquide de lavage 70 est aspiré par le tuyau 182. Le liquide est acheminé dans la pompe 20 par le tuyau 181 puis est refoulé de celle-ci par le tuyau 180, le tuyau 178 et le tuyau 171 afin d'atteindre le dispositif de filtration 100. Durant l'introduction du liquide de lavage dans le dispositif de filtration 100, on réalise un ou plusieurs cycles de centrifugation comme décrit plus haut pour séparer les matières polluantes. L'évacuation du liquide de lavage contenant les matières polluantes (eau, sang, huile etc.) du tissu adipeux est réalisé par actionnement de la pompe d'aspiration 60 et placement des modules de pincement 41, 43 et 48 dans la position d'ouverture par l'unité de commande 10. Dans cette configuration, le liquide de lavage contenant les matières polluantes est aspiré par le port d'évacuation 1131 du dispositif de filtration 100 puis circule dans les tuyaux 173, 174 et 176 afin d'être acheminé dans une poubelle 61 présente en amont de la pompe d'aspiration 60. Le placement du module 48 dans la position d'ouverture permet la mise à l'air du dispositif de filtration et facilite ainsi l'évacuation du liquide de lavage contenant les matières polluantes. Le traitement de purification du tissu adipeux avec le dispositif à usage unique de l'invention est alors achevé.

Le dispositif de vannes à pincement 40 permet de contrôler sélectivement la circulation dans les tuyaux tout en facilitant le montage et le démontage des tuyaux lors du remplacement du dispositif de filtration à usage unique.

Tout autre dispositif permettant d'empêcher sélectivement la circulation de matière dans les tuyaux pourra être utilisé pour réaliser les différentes séquences décrites précédemment.

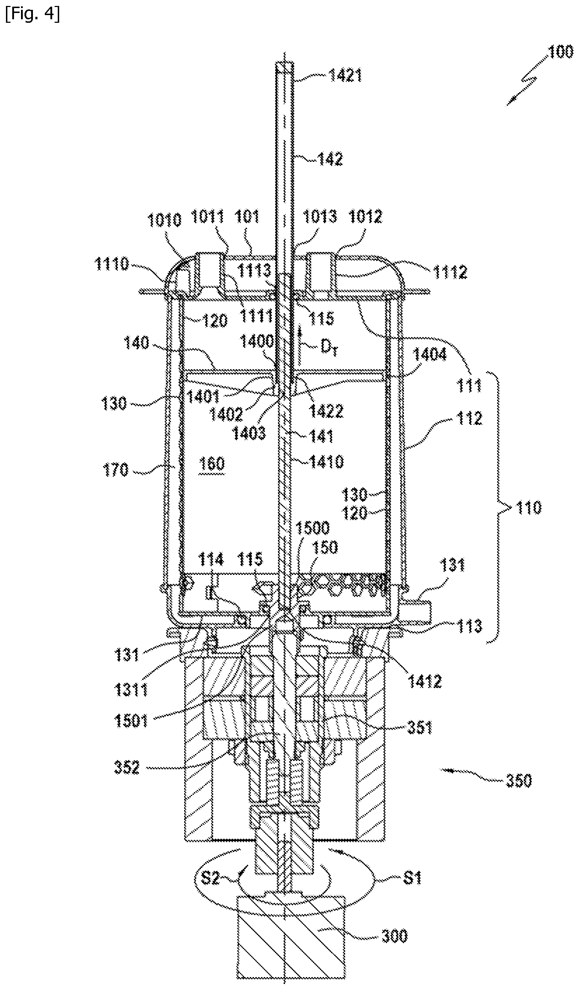

De manière optionnelle, le dispositif de purification peut en outre comprendre un plateau de collecte mobile à l'intérieur de la chambre de centrifugation. Comme illustré dans l'exemple des figures 3 et 4, le dispositif de purification 100 comprend en outre un plateau de collecte 140 qui est mobile en translation dans une direction D_{T} suivant l'axe X du filtre 120. Plus précisément, le dispositif de purification 100 comprend une tige filetée 141 qui s'étend verticalement à l'intérieur de la chambre de centrifugation 160 suivant l'axe X du filtre 120. L'extrémité inférieure 1412 de la tige filetée est reliée au moteur électrique 10 par l'intermédiaire d'un guide 150 et de l'embrayage bidirectionnel 20. Le guide 150 comprend un premier logement 1500 dans lequel l'extrémité inférieure 1412 est fixée. Une portion inférieure 1501 du guide 150 comprend un deuxième logement 1502 qui coopère avec une extrémité libre 3520 d'un deuxième arbre 352 de l'embrayage bidirectionnel 350 lorsque le dispositif de filtration à usage unique 100 est monté sur le dispositif d'entraînement en rotation 30 (figure 6).

Le deuxième arbre 352 est en prise avec le moteur électrique 300 que lorsque celui-ci transmet un mouvement de rotation suivant un deuxième sens de rotation S₂ opposé au premier sens de rotation S₁ utilisé pour la centrifugation. Lorsque le moteur électrique 300 tourne suivant le premier sens de rotation S₁, aucun mouvement de rotation n'est transmis par l'embrayage bidirectionnel 350 au guide 150 auquel est relié la tige filetée 141. De même, lorsque le moteur 300 tourne suivant le deuxième sens de rotation S₂, aucun mouvement de rotation n'est transmis par l'embrayage bidirectionnel 350 au plateau rotatif 131 et, par conséquent, au filtre 120. Le plateau de collecte 140 comporte sur sa face supérieure une ouverture centrale 1400 prolongée par un col 1401 qui s'étend à partir de la face inférieure du plateau 140. Le col 1401 comprend une portion 1402 comportant un taraudage 1403 qui coopère avec un filetage 1411 de la tige filetée 141. Ainsi, lorsque que la tige filetée est entrainée en rotation suivant le deuxième sens de rotation S₂, le plateau de collecte 140 s'élève dans la chambre de centrifugation 160 suivant la direction D_{T}.

Le bord périphérique 1404 du plateau de collecte 140 est en vis-à-vis de la paroi interne du filtre 120. Ainsi, lorsque le plateau de collecte 140 est déplacé en translation verticale suivant la direction D_{T}, celui-ci joue un rôle de piston qui permet de racler la paroi interne du filtre de manière à collecter un maximum de tissu adipeux. La translation verticale du plateau 140 permet également de placer le tissu adipeux collecté au plus près du capot 111 et de faciliter, par conséquent, son prélèvement pour réimplantation.

Selon un aspect particulier, la tige filetée 141 peut être logée dans une gaine de protection 142. La gaine de protection 142 qui s'étend entre une extrémité supérieure 1421 et une extrémité inférieure 1422 permet d'éviter à la tige filetée 141 d'entrer en contact avec le tissu adipeux qui en s'accumulant au niveau du taraudage 1403 du plateau de collecte 140 peut bloquer le déplacement de celui-ci.

La figure 4 montre le dispositif de purification 100 après actionnement du moteur électrique 10 dans le deuxième sens de rotation S₂ permettant d'entraîner en rotation la tige filetée 141. La rotation de la tige filetée 141 entraîne la translation verticale du plateau de collecte 140 suivant la direction D_{T}.

L'extrémité inférieure 1422 de la gaine de protection est fixée dans l'ouverture 1400 du plateau de collecte 140. Afin de permettre le mouvement de la gaine de protection 142 lors du déplacement du plateau 140, le capot 111 et le couvercle 101 comportent respectivement une ouverture 1113 et une ouverture 1013 au travers desquelles la gaine 142 coulisse. Un joint 115 est présent autour de l'ouverture 1113 afin de préserver l'étanchéité en sommet de la chambre de centrifugation.

Le plateau de collecte peut également être utilisé dans l'étape de réintroduction du tissu adipeux purifié dans le corps du patient. Dans ce cas, le plateau de collecte joue un rôle de piston qui pousse le tissu adipeux purifié vers la partie supérieure de la chambre de centrifugation au plus près du port 1111. L'unité de commande 10 place les modules de pincement 44 et 45 dans la position d'ouverture autorisant la circulation du tissu adipeux dans les tuyaux 171, 178 et 179 et les modules de pincement 41, 42, 43, 46, 47 et 48 dans la position de fermeture. Le tissu adipeux purifié peut être ainsi acheminé depuis la chambre de centrifugation jusqu'à la canule d'injection 55.

Dans un autre exemple de réalisation, le filtre est en un matériau rigide autoporteur. Le filtre peut être notamment réalisé à partir d'un feuillard métallique dans lequel des trous sont réalisés par laser, jet d'eau ou découpe chimique afin de former un crible, une grille métallique tissée de fils ou des billes agglomérées par frittage de poudres métalliques ou céramiques. Dans ce cas, l'élément de rigidification 130 n'est plus nécessaire et c'est le filtre autoporteur qui est directement en prise avec le plateau rotatif 131.

## Revendications

1. Dispositif de filtration à usage unique (100) pour la purification d'un tissu adipeux comprenant au moins :
- une enceinte étanche (110),
- un filtre (120) présent dans l'enceinte étanche, ledit filtre délimitant une chambre de centrifugation (160) pour un tissu adipeux,
- des tuyaux (170-183) reliés à l'enceinte étanche,
- un plateau rotatif (131) en liaison avec le filtre (120), le plateau rotatif étant configuré pour coopérer avec un dispositif d'entraînement en rotation (30),
- un plateau de collecte (140) présent dans la chambre de centrifugation (160), le plateau de collecte étant mobile en translation suivant un axe de rotation du filtre.

2. Dispositif selon la revendication 1, comprenant en outre un élément de rigidification (130) présent entre l'enceinte étanche (110) et le filtre (120).

3. Dispositif selon la revendication 1, dans lequel le filtre est en un matériau rigide autoporteur.

4. Dispositif selon la revendication 3, comprenant en outre une tige filetée (141) s'étendant dans la chambre de centrifugation (160) et coopérant avec une portion taraudée (1403) du plateau de collecte (140), la tige filetée étant configuré pour coopérer avec un moyen d'entraînement en rotation.

5. Dispositif selon la revendication 4, comprenant en outre une gaine de protection (142) entourant la tige filetée (141).

6. Machine (1) pour la purification d'un tissu adipeux, la machine comprenant au moins :
- un dispositif de filtration à usage unique (100) selon l'une quelconque des revendications 1 à 5,
- un dispositif d'entraînement en rotation (30) sur lequel le dispositif de filtration à usage unique (100) est fixé de façon amovible, le dispositif d'entraînement en rotation comprenant un moteur rotatif (300) relié au dispositif de filtration à usage unique,
- une pompe d'aspiration (60) reliée au dispositif de filtration à usage unique,
- une pompe de circulation (20) reliée au dispositif de filtration à usage unique,
- un réservoir de liquide de lavage (70) relié au dispositif de filtration à usage unique,
- une unité de commande (10) configurée pour commander le dispositif d'entraînement en rotation (30), la pompe d'aspiration (60) et la pompe de circulation (20).

7. Machine selon la revendication 6, comprenant en outre dispositif de vannes à pincement (40) coopérant avec des tuyaux du dispositif de filtration à usage unique (100), l'unité de commande (10) étant configurée pour commander ledit dispositif de vanne à pincement de manière à empêcher sélectivement la circulation dans un ou plusieurs tuyaux.

8. Machine selon la revendication 6 ou 7, dans laquelle le dispositif d'entraînement en rotation (30) comprend une bride stator (310), l'enceinte étanche (110) du dispositif de filtration à usage unique (100) comprenant des moyens de fixation amovible aptes à bloquer en rotation ladite enceinte étanche sur la bride stator.

## Patentansprüche

1. Filtrationsvorrichtung (100) zur einmaligen Verwendung für die Reinigung eines Fettgewebes, umfassend zumindest:
- eine dichte Umhüllung (110),
- einen Filter (120), der in der dichten Umhüllung vorhanden ist, wobei der Filter eine Zentrifugalkammer (160) für ein Fettgewebe begrenzt,
- Schläuche (170-183), die mit der dichten Umhüllung verbunden sind,
- eine drehende Platte (131) in Verbindung mit dem Filter (120), wobei die drehende Platte dazu konfiguriert ist, mit einer Vorrichtung zum Drehantrieb (30) zusammenzuwirken,
- eine Sammelplatte (140), die in der Zentrifugalkammer (160) vorhanden ist, wobei die Sammelplatte translatorisch entlang einer Drehachse des Filters beweglich ist.

2. Vorrichtung nach Anspruch 1, ferner umfassend ein Versteifungselement (130), das zwischen der dichten Umhüllung (110) und dem Filter (120) vorhanden ist.

3. Vorrichtung nach Anspruch 1, wobei der Filter aus einem steifen, selbsttragenden Material besteht.

4. Vorrichtung nach Anspruch 3, ferner umfassend eine Gewindestange (141), die sich in die Zentrifugalkammer (160) erstreckt und mit einem Gewindeabschnitt (1403) der Sammelplatte (140) zusammenwirkt, wobei die Gewindestange dazu konfiguriert ist, mit einer Einrichtung zum Drehantrieb zusammenzuwirken.

5. Vorrichtung nach Anspruch 4, ferner umfassend eine Schutzhülle (142), welche die Gewindestange (141) umgibt.

6. Maschine (1) für die Reinigung eines Fettgewebes, wobei die Maschine zumindest umfasst:
- eine Filtrationsvorrichtung (100) zur einmaligen Verwendung nach einem der Ansprüche 1 bis 5,
- eine Vorrichtung zum Drehantrieb (30), auf welcher die Filtrationsvorrichtung (100) zur einmaligen Verwendung nicht abnehmbar befestigt ist, wobei die Vorrichtung zum Drehantrieb einen drehenden Motor (300) umfasst, der mit der Filtrationsvorrichtung zur einmaligen Verwendung verbunden ist,
- eine Absaugpumpe (60), die mit der Filtrationsvorrichtung zur einmaligen Verwendung verbunden ist,
- eine Zirkulationspumpe (20), die mit der Filtrationsvorrichtung zur einmaligen Verwendung verbunden ist,
- einen Behälter für Waschflüssigkeit (70), der mit der Filtrationsvorrichtung zur einmaligen Verwendung verbunden ist,
- eine Steuereinheit (10), die dazu konfiguriert ist, die Vorrichtung zum Drehantrieb (30), die Absaugpumpe (60) und die Zirkulationspumpe (20) zu steuern.

7. Maschine nach Anspruch 6, umfassend ferner eine Vorrichtung mit Quetschventilen (40), die mit Schläuchen der Filtrationsvorrichtung (100) zur einmaligen Verwendung zusammenwirkt, wobei die Steuereinheit (10) dazu konfiguriert ist, die Vorrichtung mit Quetschventilen auf solche Weise zu steuern, dass die Zirkulation in einem oder mehreren Schläuchen selektiv verhindert wird.

8. Maschine nach Anspruch 6 oder 7, in welcher die Vorrichtung zum Drehantrieb (30) einen Statorflansch (310) umfasst, wobei die dichte Umhüllung (110) der Filtrationsvorrichtung (100) zur einmaligen Verwendung Einrichtungen zur nicht abnehmbaren Befestigung umfasst, die dazu geeignet sind, die dichte Umhüllung drehfest auf dem Statorflansch zu blockieren.

## Claims

1. A disposable filtration device (100) for purifying adipose tissue, comprising at least:
- a sealed enclosure (110);
- a filter (120) present in the sealed enclosure, said filter delimiting a centrifugation chamber (160) for adipose tissue;
- pipes (170-183) connected to the sealed enclosure;
- a rotary plate (131) linked to the filter (120), the rotary plate being configured to cooperate with a rotary drive device (30)
- a collector plate (140) present in the centrifugation chamber (160), the collector plate being able to move in translation along the axis of rotation of the filter.

2. The device according to claim 1, further comprising a stiffening element (130) present between the sealed enclosure (110) and the filter (120).

3. The device according to claim 1, wherein the filter is made of a rigid self-supporting material.

4. The device according to claim 3, further comprising a threaded rod (141) extending in the centrifugation chamber (160) and cooperating with a tapped portion (1403) of the collector plate (140), the threaded rod being configured to cooperate with a rotary drive means.

5. The device according to claim 4, further comprising a protective sleeve (142) surrounding the threaded rod (141).

6. A machine (1) for purifying adipose tissue, the machine comprising at least:
- a disposable filtration device (100) according to any one of claims 1 to 5,
- a rotary drive device (30) on which the disposable filtration device (100) is removably fixed, the rotary drive device comprising a rotary motor (300) connected to the disposable filtration device,
- a suction pump (60) connected to the disposable filtration device,
- a circulation pump (20) connected to the disposable filtration device,
- a reservoir of washing liquid (70) connected to the disposable filtration device,
- a control unit (10) configured to control the rotary drive device (30), the suction pump (60) and the circulation pump (20).

7. The machine according to claim 6, further comprising a pinch valve device (40) cooperating with pipes of the disposable filtration device (100), the control unit (10) being configured to control said pinch valve device so as to selectively prevent the circulation in one or more pipes.

8. The machine according to claim 6 or 7, wherein the rotary drive device (30) comprises a stator flange (310), the sealed enclosure (110) of the disposable filtration device (100) comprising removable fixation means able to lock the rotation of said sealed enclosure on the stator flange.
